# EUROPEAN PATENT APPLICATION

(11) **EP 2 345 719 A1**
(43) Date of publication of application: **20.07.2011**
(21) Application number: 10000432.4
(22) Date of filing: 18.01.2010
(51) Int. Cl.: C12N 15/10

(54) **Method for isolating small RNA**

(71) Applicant: Qiagen GmbH, 40724 Hilden (DE)
(72) Inventor: Voss, Thorsten, Dr., 51377 Leverkusen (DE); Wyrich, Ralf, Dr., 41516 Grevenbroich (DE)
(74) Representative: Roth, Carla

(57) **Abstract**

A method for isolating small RNA from a sample is provided, the method comprising binding the RNA to silica particles by contacting the sample with
a) at least one alcohol,
b) at least one chaotropic salt comprising a chaotropic anion selected from the group consisting of trichloroacetate, perchlorate and trifluoroacetate and
c) silica particles
and separating the bound RNA from the rest of the sample.

The present invention also provides compositions and kits to efficiently isolate small RNA molecules from samples, in particular biological samples such as blood, blood products tissue and body fluids.

## Description

The work leading to this invention has received funding from the European Community's Seventh Framework Programme *(FP7*/*2007-2013)* under *grant agreement* n° 222916.

The present invention pertains to methods, compositions and kits to isolate small RNA molecules from samples, in particular biological samples such as tissue and body fluids.

The study of small RNAs, i.e. RNA molecules in the order of 250 nucleotides or less from various tissues and many organisms, as well as from cultures of cells, is an area of extreme interest and promises to remain one for the future. Small RNAs include *inter alia* micro RNAs (miRNA) and small interfering RNA molecules both of which can have a powerful effect on the expression of a gene. Furthermore, also other small nuclear and small nucleolar RNAs (e.g. snRNAs and snoRNAs) involved in mRNA and rRNA processing are of importance.

With the increasing interest in respective small RNA molecules, the standard isolation procedures have been modified to facilitate the isolation of small RNAs. This, as the known protocols used as standard to isolate DNA or total RNA are usually not ideal for isolating small RNA molecules because the small RNA molecules are often not effectively captured and eluted during the isolation process. Therefore, total RNA isolated from samples using standard procedures usually does not comprise sufficient amounts of small RNA molecules. Thus, there is a need for improved techniques for the efficient isolation of small RNA molecules either alone or as a portion of total RNA.

Methods that have been optimized for the isolation of small RNA molecules often rely on phenol and chloroform extraction and stepwise alcohol fractionation. A major draw back of methods involving the use of phenol and chloroform is *inter alia* that these chemicals pose potential health risks. In addition, they are also ineffective with certain biological materials. Furthermore, phase separation and alcohol fractionation are laborious and time consuming, making them incompatible with high-throughput and automation demands.

Furthermore, improved methods for isolating small RNAs have been developed which involve the use of chaotropic agents, high concentrations of alcohol and silica membranes for the isolation of total RNA. Total RNA isolated with these protocols also comprises small RNAs as a portion. These methods are thus useful to efficiently isolate small RNA molecules. Commercially available products which are based on these methods are for example the PAXgene Blood miRNA kit, sold by QIAGEN. This kit allows the efficient isolation of small RNAs from samples. However, even though this method is very efficient in isolating small RNAs, it has the drawback that isolation is performed by means of a silica membrane. Isolation procedures using silica membranes are often time consuming and also render it difficult to isolate the small RNA by means of a flexible and dedicated automated process in a high throughput manner, e.g. by using a robotic system. Furthermore, it is desirous to be able to also process larger sample volumes. In particular, it is desirous to isolate small RNAs efficiently from complex biological samples, such as whole blood and blood products.

Therefore, there is a need for improved techniques for the efficient isolation of small RNA molecules from samples, in particular biological samples, which preferably also allow the processing by automated systems.

### SUMMARY OF THE INVENTION

The present invention provides methods to rapidly and efficiently isolate small RNA molecules from samples by using silica particles. The use of silica particles, in particular magnetic silica particles, allows the rapid, flexible and also automated high throughput isolation of small RNA molecules from samples. The inventors identified special binding conditions that allow the efficient isolation of small RNAs even from complex samples when using silica particles.

Therefore, according to one aspect of the present invention, a method for isolating small RNA from a sample is provided wherein the method comprises binding the RNA to silica particles by contacting the sample with
a) at least one alcohol,
b) at least one chaotropic salt comprising a chaotropic anion selected from the group consisting of trichloroacetate, perchlorate and trifluoroacetate and
c) silica particles
   and separating the bound RNA from the rest of the sample.
   It has been found that this method allows the efficient and rapid isolation of small RNA from samples with good yield. The small RNA is preferably obtained as part of total RNA.

According to a further aspect of the present invention a kit for isolating small RNA from a sample is provided, comprising
a) at least one alcohol,
b) at least one chaotropic salt comprising a chaotropic anion selected from the group consisting of trichloroacetate, perchlorate and trifluoroacetate and
c) silica particles.

A further aspect of the present invention encompasses a composition for binding small RNA to silica particles comprising
a) at least one alcohol,
b) at least one chaotropic salt comprising a chaotropic anion selected from the group consisting of trichloroacetate, perchlorate and trifluoroacetate and
c) silica particles.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only. Various changes and modifications within the spirit and scope of the disclosed invention will become readily apparent to those skilled in the art from reading the following.

### DETAILED DESCRIPTION OF THE INVENTION

It has been found that silica particles such as for example magnetic silica particles differ considerably in their binding characteristics from silica membranes. Therefore, the binding conditions established for silica membranes are in particular for complex samples such as whole blood samples not suitable for binding small RNA efficiently and particularly in comparable amounts to silica particles. This makes the isolation and purification of small RNA with acceptable yields challenging when using silica particles.

Therefore, it was necessary to find suitable binding conditions which allow the efficient isolation of small RNA when using silica particles such as in particular magnetic silica particles as nucleic acid binding carrier. The use of silica particles and in particular magnetic silica particles has advantages regarding the processing of the particles for example in automated processes.

It was now surprisingly found that by using specific chaotropic anions it is possible to bind small RNAs effectively to silica particles. When using specific chaotropic anions, binding of the small RNAs is considerably improved compared to regular binding conditions which comprise commonly used chaotropic agents such as for example guanidiniumisothiocyanate or guanidinumhydrochloride.

Therefore, according to a first aspect of the present invention, a method for isolating small RNA from a sample is provided, said method comprising binding the RNA to silica particles by contacting a sample with
a) at least one alcohol,
b) at least one chaotropic salt comprising a chaotropic anion selected from the group consisting of trichloroacetate, perchlorate and trifluoroacetate and
c) silica particles
and separating the bound RNA from the rest of the sample.

It was surprisingly found that the respective binding conditions allow the efficient binding of small RNA to silica particles, which are preferably magnetic silica particles to simplify the processing of the silica particles via a magnet, thereby allowing besides manual processing also the easy processing by using robotic systems.

According to one embodiment, said chaotropic anion selected from the group consisting of trichloroacetate, perchlorate and trifluoroacetate is present in the binding composition in a concentration of at least 0,05M up to the saturation limit. According to one embodiment, the concentration lies in a range from 0,05M to 4M. According to one embodiment, the concentration lies in a range from 0,05M to 3M and preferably, in a range from 0,05M to 2M. According to a very preferred embodiment, the concentration of said chaotropic anion selected from the group consisting of trichloroacetate, perchlorate and trifluoroacetate lies in a range from 0,1M to 1M. Most preferred, the concentration of said chaotropic anion is less than 1M and lies in a range from 0,1 M to 0,9M or 0,1 M to 0,8M. Accordingly, the chaotropic salt comprising a chaotropic anion selected from the group consisting of trichloroacetate, perchlorate and trifluoroacetate is preferably present in the binding composition in the concentrations described above for the chaotropic anion according to the present invention in particular when the salt comprises a monovalent cation. As cation, an alkali metal ion such as preferably sodium can be used. Therefore, according to one embodiment the chaotropic salt is selected from the group consisting of sodium perchlorate, sodium trichloroacetate and sodium trifluoroacetate. As is shown in the examples, the respective chaotropic salts are particularly well suitable for the isolation of small RNA when using silica particles as nucleic acid binding carrier. Preferably, sodium trichloroacetate is present in the binding composition in a concentration of 0,1M to less than 1 M, preferably in a concentration of 0,1M to 0,7M. If desired, also other chaotropic salts such as e.g. the commonly used guanidinium salts can be used in addition to the chaotropic salt comprising a chaotropic anion selected from the group consisting of trichloroacetate, perchlorate and trifluoroacetate.

For isolating small RNA by using silica particles, it is beneficial to use an alcohol concentration of ≥ 40% v/v, preferably ≥ 40% v/v to ≤ 80% v/v during binding and thus in the binding composition. Most preferred is an alcohol concentration of ≥ 50% v/v to ≤ 65% v/v during binding. Also mixtures of alcohol can be used.

As alcohol, short chained branched or unbranched alcohols with preferably one to 5 carbon atoms can be used. Examples are methanol, ethanol, propanol, isopropanol and butanol. The alcohol is preferably selected from isopropanol and ethanol, particularly well suitable is isopropanol.

Optionally, one or more detergents can be comprised in the binding composition. Preferably, ionic and/or non-ionic detergents are used as detergent. Preferably, a non-ionic detergent is used in a concentration of at least 5%. Said detergent can be added, e.g., with the binding buffer.

Furthermore, a biological buffer can be used for binding. Non-limited examples of biological buffers include but are not limited to HEPES, MES, MOPS, TRIS, BIS-TRIS Propane and others. Preferably, a Tris buffer is used. Therefore, according to one embodiment, the sample is contacted with a binding buffer which comprises at least one alcohol, at least one chaotropic salt comprising a chaotropic anion selected from the group consisting of trichloroacetate, perchlorate and trifluoroacetate and a biological buffer, preferably Tris. Optionally, the binding buffer may also comprise a detergent as is described above. However, the components can also be added separately to the sample to form a suitable binding composition. Preferably, the binding buffer buffers in a range that includes pH 8. According to one embodiment, the binding buffer has a pH in the range from pH 7,0 to 9, preferably 7,5 to 8,5; most preferred the binding buffer has a pH of 8.

The silica particles can be used in form of beads and preferably have a particle size of about 0,02 to 30 µm, more preferred 0,05 to 15 µm and most preferred of 0,1 to 10 µm. To ease the processing of the nucleic acid binding carrier, preferably magnetic silica particles are used. The magnetic silica particles may e.g. be ferrimagnetic, ferromagnetic, paramagnetic or superparamagnetic. Suitable magnetic silica particles are for example described in WO 01/71732, WO 2004/003231 and WO 2003/004150. Other magnetic silica particles are also known from the prior art and are e.g. described in WO 98/31840, WO 98/31461, EP 1 260 595, WO 96/41811 and EP 0 343 934 and also include for example magnetic silica glass particles.

As it is outlined above, the method of the present invention is particularly suitable to retrieve small RNAs from a sample. Typically, small RNA molecules are less than about 250 nucleotides, preferably less than 200 nucleotides in length. Both prokaryotic and eukaryotic cells contain a plurality of different sized RNA molecules. RNA molecules with lengths greater than about 200 or 250 nucleotides include long noncoding RNA (IncRNA)/large intergenic noncoding RNA (lincRNA), messenger RNA (mRNA), 16S/18S ribosomal RNA (rRNA), and 23S/28S rRNA. Small RNA molecules with lengths less than about 200 to 250 nucleotides include 5.8S rRNA, 5S rRNA, transfer RNA (tRNA), small nuclear RNA (snRNA), small nucleolar RNA (snoRNA); micro RNA (miRNA), small interfering RNA (siRNA), trans-acting siRNA (tasiRNA), repeat-associated siRNA (rasiRNA), small temporal RNA (stRNA), tiny non-coding RNA (tncRNA), small scan RNA (scRNA), and small modulatory RNA (smRNA). The small RNAs can be obtained as a portion of total RNA and thus are included in a substantial amount in the total RNA isolated from a sample.

The sample can be of any kind and therefore can also be an *in vitro* sample comprising small RNA molecules. Preferably, the sample is a biological sample derived from a human, animal, plant, bacteria or fungi. Preferably, the sample is selected from the group consisting of cells, tissue, bacteria, virus and body fluids such as for example blood, blood products such as buffy coat, plasma and serum, urine, liquor, sputum, stool, CSF and sperm, epithelial swabs, biopsies, bone marrow samples and tissue samples, preferably organ tissue samples such as lung and liver. Preferably, the sample is selected from whole blood and blood products such as buffy coat, serum or plasma.

According to one embodiment, which is particularly preferred when RNA is isolated from a biological sample such as whole blood or blood products, the nucleic acids are stabilised preferably immediately after the biological sample has been taken from its natural environment in order to preserve the status quo of the sample and in particular the transcription pattern. This is particularly beneficial in the medical and diagnostic field.

Thus, according to one embodiment, the biological sample is mixed with a nucleic acid storage stabilization composition for stabilizing nucleic acids in said biological sample prior to isolating the nucleic acids from the sample. According to one embodiment, said stabilization composition comprises
a) a cationic compound of the general formula:

   Y⁺R₁R₂R₃R₄X⁻

   wherein Y represents nitrogen or phosphor, preferably nitrogen
   R₁R₂R₃ and R₄ independently, represent a branched or unbranched C₁-C₂₀-alkyl group, a C₆-C₂₀-aryl group and/or a C₆-C₂₆ aralkyl group;
   X' represents an anion of an inorganic or organic, mono- or polybasic acid; and
b) at least one proton donor, wherein the proton donor is preferably present in the composition in a concentration of above 50 mM to saturation and wherein the proton donor is preferably selected from the group consisting of saturated aliphatic monocarboxylic acids, unsaturated alkenyl-carboxylic acids, saturated and/or unsaturated aliphatic C₂-C₆-dicarboxylic acids, aliphatic hydroxyl-di- and tricarboxylic acids, aliphatic ketocarboxylic acids, amino acids or the inorganic acids or the salts thereof, on their own or in combination.

Preferably, R₁ denotes a higher alkyl group with 12, 14 or 16 carbon atoms and R₂, R₃ and R₄ each represent a methyl group.

Preferably, the anion X⁻ represents an anion of hydrohalic acids or anions of mono- or dibasic organic acids, most preferred the anion X' is selected from the group consisting of bromide, chloride, phosphate, sulphate, formate, acetate, propionate, oxalate, malonate, succinate or citrate.

Preferably, the proton donor is selected from the group consisting of saturated aliphatic monocarboxylic acids, unsaturated alkenyl-carboxylic acids, saturated and/or unsaturated aliphatic C₂-C₆ -dicarboxylic acids, aliphatic ketocarboxylic acids, amino acids or the inorganic acids or the salts thereof, and combinations thereof. Preferably, the aliphatic monocarboxylic acid comprises a C₁-C₆-alkyl-carboxylic acid selected from the group consisting of acetic acid, propionic acid, n-butyric acid, n-valeric acid, isovaleric acid, ethyl-methyl-acetic acid (2-methyl-butyric acid), 2,2-dimethylpropionic acid (pivalic acid), n-hexanoic acid, n-octanoic acid, n-decanoic acid or n-dodecanoic acid (lauric acid) or mixtures thereof. Preferably, the aliphatic alkenyl-carboxylic acid is selected from the group consisting of acrylic acid (propenoic acid), methacrylic acid, crotonic acid, isocrotonic acid or vinylacetic acid or mixtures thereof. Preferably, the saturated aliphatic C₂-C₆-dicarboxylic acid is selected from the group consisting of oxalic acid, malonic acid, succinic acid, glutaric acid or adipic acid or mixtures thereof. Most preferred, the aliphatic dicarboxylic acid is oxalic acid or succinic acid or mixtures thereof. Preferably, the aliphatic hydroxy-di- and -tricarboxylic acids are selected from the group consisting of tartronic acid, D-(+), L-(-) or DL-malic acid, (2R, 3R)-(+)-tartaric acid, (2S, 3S)-(-)-tartaric acid, meso-tartaric acid and citric acid or mixtures thereof. Most preferred, the unsaturated dicarboxylic acid is maleic and/or fumaric acid or mixtures thereof. Preferably, the unsaturated tricarboxylic acid is aconitic acid. Preferably, the aliphatic ketodicarboxylic acids are mesoxalic acid or oxaloacetic acid, or mixtures thereof. Preferably, the amino acids are selected from the group consisting of aminoacetic acid (glycine), alpha -aminopropionic acid (alanine), alpha -amino-isovaleric acid (valine), alpha -amino-iso-caproic acid (leucine) and alpha -amino- beta - methylvaleric acid (isoleucine), or mixtures thereof.

Preferably, the stabilising composition is present in an aqueous solution. Preferably, the cationic compound is comprised in a concentration in the range from 0.01 weight percent to 15 weight percent.

Suitable stabilising solutions are also described in detail e.g. in US 7,270,953, herein incorporated by reference.

Complex samples that are stabilised as is described above are a particular challenge for isolating nucleic acids due to the additives in the stabilising solution. The method of the present invention overcomes these difficulties and allows the isolation of small RNA with a good yield, in particular as part of total RNA from respectively stabilised samples and in particular complex biological samples such as whole blood and blood products such as buffy coat, serum and/or plasma or tissue samples, in particular organ tissue samples e.g. obtained from lung or liver. Therefore, the method of the present invention is particularly useful in the medical and in particular in the diagnostic field.

Furthermore, it was surprisingly found that the method of the present invention allows the isolation of RNA, including the small RNAs, also from large sample volumes also when being confronted with complex samples such as whole blood, blood products or tissue samples. Thus, complex samples having a volume of at least 1ml, at least 2ml and preferably at least 2,5ml can be efficiently processed with the method of the present invention.

Depending on the used sample, it can be useful to pretreat the sample prior to binding the RNA to the silica particles. Therefore, according to one embodiment, at least a portion of the sample is pretreated prior to binding by the addition of at least one lysis agent. Preferably, a protease such as proteinase K is used for lysis and a chaotropic agent, such as guanidiniumhydrochloride, guanidiniumthiocyanate, guanidiniumisothiocyanate, sodium iodide and/or sodium perchlorate. Other suitable methods for lysis include for example the use of a detergent such as for example SDS, LiDS or sarkosyl. However, also other suitable lysis methods known in the prior art can be used. As respective methods are known in the prior art, they do not need further description here. Preferably, lysis is performed in a buffer at neutral pH using proteinase K and a chaotropic agent.

According to one embodiment, non-target nucleic acids are at least partly removed after lysis by the addition of a suitable nucleic acid binding carrier under conditions wherein mainly the non-target nucleic acids are bound to the nucleic acid binding carrier. For this purpose, again silica particles can be used. Usually, the non-target nucleic acid will be DNA when RNA is isolated from the biological sample. Suitable methods for removing respective non-target nucleic acids are for example described in EP 0 880 537 and WO 95/21849.

According to one embodiment for isolation of RNA, the supernatant that is obtained after removal of the non-target nucleic acid is contacted with silica particles, at least one alcohol and at least one chaotropic salt comprising a chaotropic anion selected from the group consisting of trichloroacetate, perchlorate and trifluoroacetate to bind the RNA including the small RNAs to the silica particles as is described above. Suitable binding conditions are described above. It is referred to the above disclosure.

According to a preferred embodiment, after binding to the silica particles, the RNA is washed at least once with one or more washing buffers. An example of a suitable washing buffer comprises at least one chaotropic agent and/or at least one alcohol. Chaotropic agents that can be used in the washing buffers include guanidiniumhydrochloride, guanidiniumthiocyanate, guanidiniumisothiocyanate and sodium iodide. Furthermore, chaotropic salts can be used which comprise a chaotropic anion selected form the group consisting of trichloroacetate, perchlorate and trifluoroacetate. Examples of respective chaotropic salts are alkali salts like sodium perchlorate, sodium trichloroacetate and sodium trifluoroacetate. As alcohol, short chained branched or unbranched alcohols with preferably one to 5 carbon atoms can be used. Examples are methanol, ethanol, propanol, isopropanol and butanol. Preferably, isopropanol and/or ethanol are used. Preferably, the washing buffer comprises at least 50% alcohol and at least 1M chaotropic salt, preferably at least 2M chaotropic salt. Furthermore, the washing buffer may comprise a detergent. Preferably, ionic and/or non-ionic detergents are used as detergent. Preferably, a non-ionic detergent is used in a concentration of at least 5%.

A further suitable washing buffer which can be used alternatively or also in addition to the washing buffer described above comprises an alcohol and a biological buffer. Suitable alcohols and biological buffers are described above. Preferably, isopropanol or ethanol, most preferred ethanol is used for this second washing step. Preferably, ethanol is used in a concentration of at least 70% v/v, preferably at least 80% v/v. The biological buffer is preferably Tris at a pH of approx. 7 to 8.

According to one embodiment, a DNase digest is performed after the first washing step(s). As the conditions for performing a DNase digest are well known in the prior art, they do not need further description here.

According to one embodiment, after the first washing step(s) and optionally the DNase digest, the silica particles and the RNA are contacted again with at least one alcohol and at least one chaotropic salt comprising a chaotropic anion selected from the group consisting of trichloroacetate, perchlorate and trifluoroacetate. For this purpose, preferably a binding buffer is used which comprises the respective agents and optionally, a detergent as is described above. This second RNA binding step ensures the tight binding of the RNA and in particular the small RNA to the silica particles and therefore, ensures a good yield. The second binding step can be performed under conditions as are described above wherein suitable concentrations and examples are described in particular for the chaotropic anion selected from the group consisting of trichloroacetate, perchlorate and trifluoroacetate, the corresponding chaotropic salts, alcohols and biological buffers. It is referred to the above disclosure.

Optionally, further washing steps can be performed after the second RNA binding step. As washing buffer, the same washing buffers as described above can be used. It is referred to the above disclosure.

If desired, the RNA can be eluted from the silica particles. Alternatively, the RNA might also be processed while still being bound to the silica particles, depending on the intended downstream application. Elution can be performed for example with classical elution buffers, such as water, buffers, in particular biological buffers such as Tris and preferably, elution solutions are used that do not interfere with the intended downstream application.

After performing the above described isolation of small RNA, optionally as part of total RNA, the small RNA can be used, respectively further processed, e.g. in amplification, modification, reverse transcription or analysis procedures.

Also provided is a kit for isolating small RNA from a sample, comprising
a) optionally at least one alcohol,
b) at least one chaotropic salt comprising a chaotropic anion selected from the group consisting of trichloroacetate, perchlorate and trifluoroacetate and
c) silica particles and
d) optionally instructions for use.

According to one embodiment, the kit comprises as alcohol a short chained branched or unbranched alcohol with preferably one to 5 carbon atoms. Examples are methanol, ethanol, propanol, isopropanol and butanol. Preferably, isopropanol and/or ethanol are included in the kit as alcohol. As described above, isopropanol is most preferred.

The kit may comprise a binding buffer comprising at least one alcohol, at least one chaotropic salt comprising a chaotropic anion selected from the group consisting of trichloroacetate, perchlorate and trifluoroacetate. Providing the alcohol and the chaotropic salt in one binding buffer has the advantage that the customer does not need to mix the respective components separately to form the binding composition. The binding buffer comprises the alcohol preferably in a concentration suitable to render a concentration of alcohol in the binding composition of approx. ≥ 40% v/v, preferably ≥, 40% v/v to ≤ 80% v/v. Most preferred the alcohol is provided in the binding buffer suitable to render an alcohol concentration of ≥ 50% v/v to ≤ 65% v/v in the binding composition. For this purpose, according to one embodiment, the binding buffer comprises alcohol in a concentration of at least 60% v/v, preferably at least 70% v/v.

Furthermore, according to one embodiment the binding buffer comprises the chaotropic salt, which comprises a chaotropic anion selected from the group consisting of trichloroacetate, perchlorate and trifluoroacetate. According to one embodiment, said chaotropic anion selected from the group consisting of trichloroacetate, perchlorate and trifluoroacetate is present in the binding buffer in a concentration suitable to obtain in the binding composition a concentration of said chaotropic anion of at least 0,05M up to the saturation limit. According to one embodiment, said chaotropic anion is present in the binding buffer in a concentration suitable to obtain in the binding composition a concentration of said chaotropic anion that lies in a range from 0,05M to 4M. According to one embodiment, said chaotropic anion is present in the binding buffer in a concentration suitable to obtain in the binding composition a concentration of said chaotropic anion that lies in a range from 0,05M to 3M, preferably, in a range from 0,05M to 2M. According to a very preferred embodiment, said chaotropic anion is present in the binding buffer in a concentration suitable to obtain in the binding composition a concentration of said chaotropic anion that lies in a range from 0,1M to 1 M. Most preferred, said chaotropic anion is present in the binding buffer in a concentration suitable to obtain in the binding composition a concentration of said chaotropic anion that is less than 1M and lies in a range from 0,1 M to 0,9M or 0,1 M to 0,8M. Accordingly, the chaotropic salt comprising a chaotropic anion selected from the group consisting of trichloroacetate, perchlorate and trifluoroacetate can be present in the binding buffer in the concentrations described above for said chaotropic anion. As cation of the chaotropic salt, an alkali metal ion such as preferably sodium can be used. Therefore, according to one embodiment, a chaotropic salt is comprised in the binding buffer that is selected from the group consisting of sodium perchlorate, sodium trichloroacetate and sodium trifluoroacetate. As is shown in the examples, the respective chaotropic salts are particularly well suitable for the isolation of small RNA as part of total RNA when using silica particles as nucleic acid binding carrier. Preferably, sodium trichloroacetate is present in the binding buffer in a concentration of 0,1M to less than 1 M. According to a preferred embodiment, the binding buffer comprises at least 0,5M of the chaotropic salt, which is preferably sodium trichloroacetate. Sodium trichloroacetate is preferably present in the binding buffer in a concentration of 0,6M. As is described above, other chaotropic salts such as e.g. the commonly used guanidinium salts can be comprised in the binding buffer in addition to the chaotropic salt comprising a chaotropic anion selected from the group consisting of trichloroacetate, perchlorate and trifluoroacetate.

Furthermore, the binding buffer may comprise a biological buffer. Details with respect to the biological buffer and suitable pH values are described above in conjunction with the method according to the present invention. It is referred to the above disclosure. Preferably, a Tris buffer is used which has a pH between 7 and 9, 8,5, preferably pH 8 to 8,5.

Preferably, magnetic silica particles are comprised in the kit. This, as the magnetic silica particles simplify the handling and thus processing of the silica particles during isolation. The use of magnetic silica particles also allows the automated processing of the isolation protocol for example via a robotic system. Suitable magnetic silica particles are described above and are also known in the prior art.

According to a further embodiment the kit may additionally or alternatively comprise a lysis buffer, preferably comprising at least one chaotropic agent. Furthermore, the kit may additionally or alternatively comprise a solution comprising a lysis enzyme such as for example a protease, preferably proteinase K.

Furthermore, the kit may comprise one or more washing buffers. Suitable washing buffers are described above; it is referred to the above disclosure.

Furthermore, the kit may additionally or alternatively, comprise an elution buffer. Suitable elution buffers are described above; it is referred to the above disclosure.

According to a further embodiment, a composition for binding small RNA to silica particles is provided which comprises
a) at least one alcohol,
b) at least one chaotropic salt comprising a chaotropic anion selected from the group consisting of trichloroacetate, perchlorate and trifluoroacetate and
c) silica particles and
d) at least one sample or portion or part of a sample comprising nucleic acids.

The advantages of using a respective binding composition for isolating small RNA by using silica particles are described in detail above. It is referred to the above disclosure.

The composition which is suitable for isolating small RNA from the sample, preferably as a portion of total RNA, preferably has one or more of the following characteristics:
According to one embodiment, it comprises as alcohol a short chained branched or
unbranched alcohol, preferably with one to 5 carbon atoms. Examples are methanol, ethanol, propanol, isopropanol and butanol. Preferably, isopropanol and/or ethanol is comprised as alcohol, most preferred isopropanol. The alcohol is according to one embodiment comprised in a concentration of approx. ≥ 40% v/v, preferably ≥ 40% v/v to s 80% v/v. Most preferred the alcohol is comprised in a concentration of ≥ 50% v/v to ≤ 65% v/v.

According to one embodiment, said chaotropic anion selected from the group consisting of trichloroacetate, perchlorate and trifluoroacetate is present in the binding composition in a concentration of at least 0,05M up to the saturation limit. According to one embodiment, the concentration lies in a range from 0,05M to 4M. According to one embodiment, the concentration lies in a range from 0,05M to 3M, preferably, in a range from 0,05M to 2M. According to a very preferred embodiment, the concentration of said chaotropic anion selected from the group consisting of trichloroacetate, perchlorate and trifluoroacetate in the binding composition lies in a range from 0,1 M to 1M. Most preferred, the concentration of said chaotropic anion is less than 1M and lies in a range from 0,1M to 0,9M or 0,1M to 0,8M. Accordingly, the chaotropic salt comprising a chaotropic anion selected from the group consisting of trichloroacetate, perchlorate and trifluoroacetate can be present in the binding composition in the concentrations described above for said chaotropic anion. The corresponding cation is preferably an alkali metal ion such as sodium. Therefore, according to one embodiment, the binding composition comprises a chaotropic salt selected from the group consisting of sodium perchlorate, sodium trichloroacetate and sodium trifluoroacetate. As is shown in the examples, the respective chaotropic salts are particularly well suitable for the isolation of small RNA when using silica particles as nucleic acid binding carrier. Preferably, sodium trichloroacetate is present in the binding composition in a concentration of 0,1M to less than 1 M, preferably in a concentration of 0,1M to 0,7M. Of course, other chaotropic salts such as e.g. the commonly used guanidinium salts can be comprised in addition to the chaotropic salt comprising a chaotropic anion selected from the group consisting of trichloroacetate, perchlorate and trifluoroacetate.

Furthermore, the composition may comprise a biological buffer which is preferably Tris. Examples of suitable biological buffers and suitable pH values are described above in conjunction with the method according to the present invention. It is referred to the above disclosure.

As is outlined above, the binding composition may also comprise a detergent; it is referred to the above disclosure for details.

The advantages and further characteristics of the respective composition were also outlined above in conjunction with the method for isolating small RNA by using silica particles. It is referred to the above disclosure.

### FIGURES

### Fig. 1

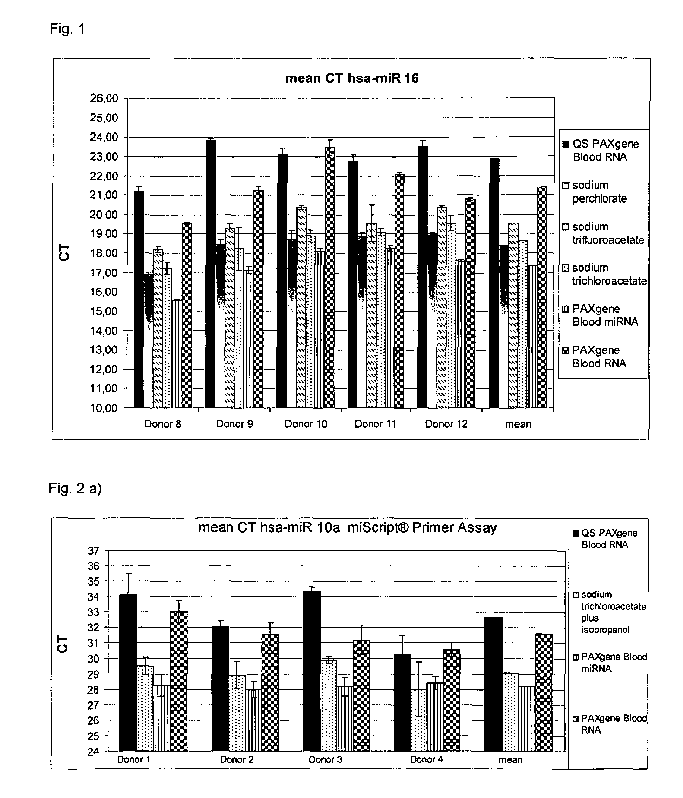

Fig. 1 shows the results of a miScript® primer assay (QIAGEN) comparing the eluates generated with the
   - QlAsymphony PAXgene Blood RNA Kit (an automated kit wherein magnetic silica particles are used as nucleic acid binding carrier that are processed by the QlAsymphony robotic system; PreAnalytiX),
   - the PAXgene Blood miRNA Kit (a dedicated kit for isolation of miRNAs using a silica membrane as nucleic acid binding carrier for isolating RNA; PreAnalytiX),
   - the PAXgene Blood RNA Kit (a general RNA isolation kit using a silica membrane as nucleic acid binding carrier; PreAnalytiX) and
   - RNA isolation method using binding buffers according to the method of the present invention containing sodium perchlorate, sodium trifluoroacetate or sodium trichloroacetate, respectively, wherein magnetic silica particles are used as nucleic acid binding carrier for isolation, here MagAttract particles (QIAGEN) processed by the QlAsymphony robotic system (QIAGEN).

For details on the performed isolation procedures, please refer to the respective description in the examples.

To analyse whether the small RNA was effectively isolated as part of the total RNA by the different compared methods, the miRNA has-miR 16 was detected in the miScript® Primer Assay. The results are shown in Fig. 1.

### Fig. 2

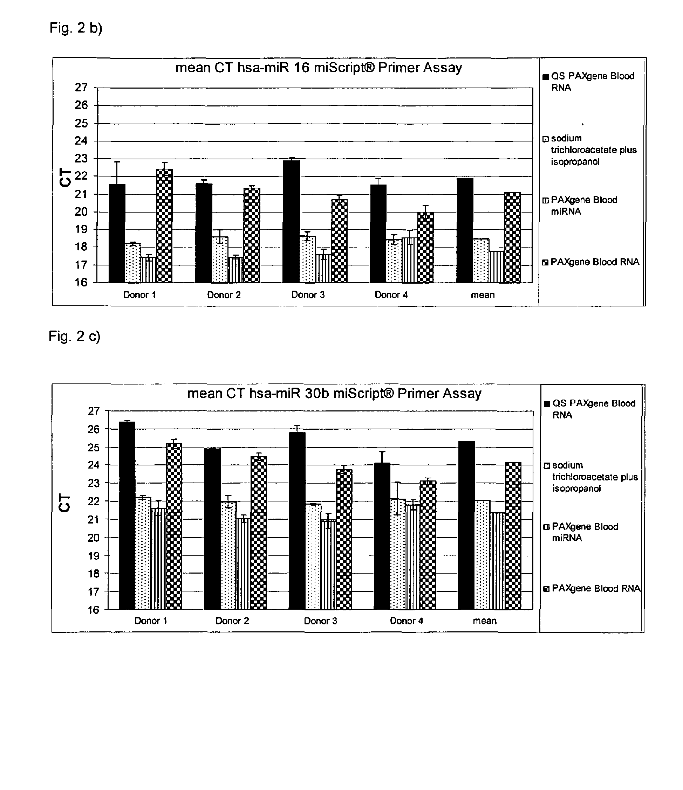

Fig. 2 a) to c) show the results of three miScript Assays (QIAGEN) comparing eluates generated with the QlAsymphony PAXgene Blood RNA Kit, the PAXgene Blood miRNA Kit, the PAXgene Blood RNA Kit and a binding buffer according to the present invention comprising sodium trichloroacetate which was used with MagAttract beads as nucleic acid binding carrier (for details on the isolation procedure of the present invention, please refer to the respective description in the examples). Here, the miRNAs hsa-miR 10a, hsa-miR 16 and hsa-miR 30b were detected.

### Fig. 3

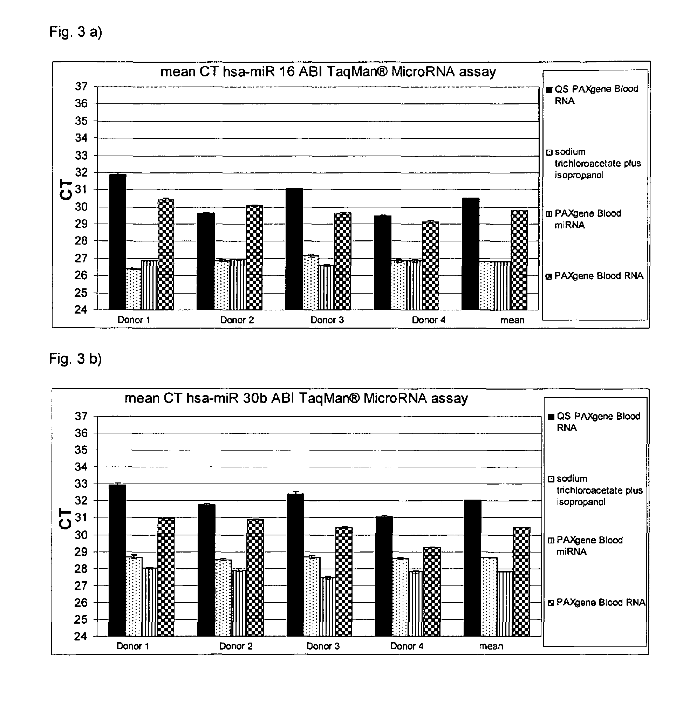

Fig. 3 a) and b) show the results of two ABI TaqMan® MicroRNA assays (Life Technologies) comparing the eluates generated with the QlAsymphony PAXgene Blood RNA Kit, the PAXgene Blood miRNA Kit, the PAXgene Blood RNA Kit and a binding buffer according to the present invention comprising sodium trichloroacetate which was used with MagAttract beads as nucleic acid binding carrier (for details of the isolation procedure of the present invention, please refer to the respective description in the examples). Here, the miRNAs has-miR 16 and hsa-miR 30b were detected.

### Fig. 4

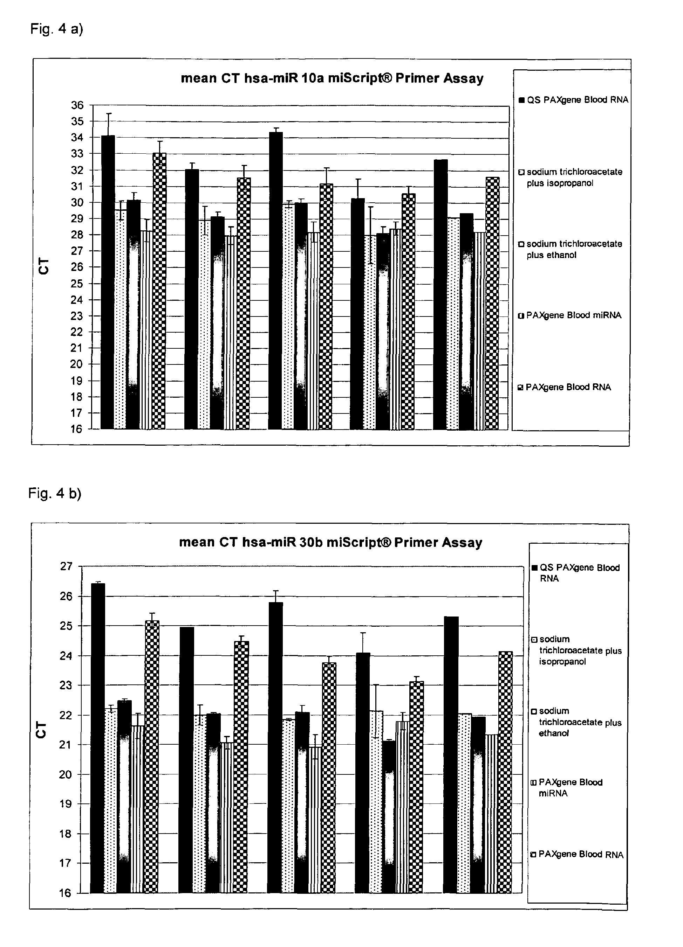

Fig. 4 a) and b) show the results of two miScript® Primer Assays (QIAGEN) comparing eluates generated with the QlAsymphony PAXgene Blood RNA Kit, the PAXgene Blood miRNA Kit, the PAXgene Blood RNA Kit and binding buffers according to the present invention comprising sodium trichloroacetate with either isopropanol or ethanol, which are used with MagAttract beads as nucleic acid binding carrier (for details of the isolation procedure of the present invention, please refer to the respective description in the examples). Here, the miRNAs hsa-miR 10a and hsa-miR 30b were detected.

### EXAMPLES

### 1. RNA isolation protocols

Total RNA was isolated from whole blood (2,5ml) from 5 different donors in PAXgene Blood RNA tubes which comprise a stabilisation solution. Suitable stabilisation solutions are also described above. The PAXgene Blood RNA tubes (PreAnalytiX) comprising the stabilised sample were further processed according to the instruction manuals for the commercially available kits QIAsymphony PAXgene Blood RNA Kit (PreAnalytiX), PAXgene Blood miRNA Kit (PreAnalytiX), the PAXgene Blood RNA Kit (PreAnalytiX) or the method according to the present invention.

The stabilised sample was processed as follows:

### a) QIAsymphony PAXgene Blood RNA (PreAnalytiX)

The detailed protocol is described in the corresponding handbook. Thus, the respective protocol is only briefly described herein:
1. The PAXgene Blood RNA tubes were centrifuged for 10 minutes at 3000 - 5000 x g using a swing-out rotor.
2. After centrifugation, the supernatant was removed by decanting. The supernatant was discarded and the pellet was saved for resuspension in step 3.
3. 300µl Buffer BR1 was added per tube. The tubes were closed and the pellet was thoroughly resuspended by vortexing. A multitube vortexer was used for resuspension at full speed for 30 seconds, respectively until the pellets were completely resuspended.
4. The closures were removed and discarded.
5. The robotic system QlAsymphony SP was used for further processing. The required reagent cartridge for the PAXgene Blood RNA isolation and consumables were loaded into the "Reagents and Consumable" drawer. Furthermore, the required elution rack was loaded into the "Eluate" drawer.
6. The samples from step 4 were placed into the appropriate sample carrier and loaded into the "Sample" drawer. Afterwards, the samples were processed by the QlAsymphony with the corresponding program. In brief, the robotic system QIAsymphony processes the probes by adding the lysis buffer BR2, proteinase K and the MagAttract beads. First, DNA is bound to the beads and removed. Afterwards, further magnetic beads are added in addition to the binding buffer QSB1. Afterwards, the complexes are pre-washed with buffer QSB1 and buffer BR4.
7. The samples are pre-eluted with buffer BR5 and remaining DNA is digested. The RNA is rebound by adding the buffer QSB2. Afterwards, further washing steps are performed, the complexes are dried and the RNA is eluted.

### b) PAXgene Blood miRNA Kit (PreAnalytiX)

The detailed protocol is described in the corresponding handbook. Thus, the respective protocol is only briefly described herein:
1. The PAXgene Blood RNA tubes were centrifuged for 10 minutes at 3000-5000 x g using a swing-out rotor.
2. The supernatant was removed by decanting or pipetting. 4 ml RNase-free water was added to the pellet and the tubes were closed.
3. The pellets were vortexed until they are visibly dissolved and centrifuged for 10 minutes at 3000-5000 x g using a swing-out rotor. The entire supernatant was removed by decanting or pipetting and discarded.
4. 350µl of the buffer BM1 was added and vortexed until the pellet was visibly dissolved.
5. The sample was pipetted into a 1.5 ml microcentrifuge tube. 300 µl buffer BM2 was added and 40µl proteinase K. These components were mixed by vortexing for 5 seconds and incubated for 10 minutes at 55°C in a shaker-incubator at 400-1400 rpm. After incubation, the temperature of the shaker-incubator was set to 65°C for use in step 20.
6. The sample was pipetted into a PAXgene Shredder spin column and placed in a 2 ml processing tube and centrifuged for 3 minutes at full speed.
7. The entire supernatant of the flow-through from the PAXgene Shredder spin column was carefully transferred to a new 1.5 ml microcentrifuge tube without disturbing the pellet in the processing tube.
8. 700µl of isopropanol (100%, purity grade p.a.) was added and mixed by vortexing.
9. 700µl of the sample was pipetted into the PAXgene RNA spin column and placed in 2 ml processing tube. After centrifugation for 1 minute at 8000-20000 x g the spin column was placed in a new 2 ml processing tube and the old processing tube containing the flow-through was discarded.
10. The remaining sample was pipetted into the PAXgene RNA spin column and centrifuged for 1 minute at 8000-20000 x g. The spin column was placed in a new 2 ml processing tube and the old processing tube was discarded.
11. 350µl Buffer BM3 was added to the PAXgene RNA spin column. The lid was closed and centrifuged for 15 seconds at 8000-20000 x g. The spin column was placed in a new 2 ml processing tube and the old processing tube containing the flow-through was discarded.
12. 10µl DNase I stock solution was added to 70 µl Buffer RDD in a 1.5 ml microcentrifuged tube.
13. The DNase I incubation mix (80µl) was pipetted directly onto the PAXgene RNA spin column membrane and incubated on the benchtop for 15 minutes.
14. 350µl Buffer BM3 was added to the PAXgene RNA spin column. The lid was closed and centrifuged for 15 seconds at 8000-20000 x g. The spin column was placed in a new 2 ml processing tube and the old processing tube containing flow-through was discarded.
15. 500µl Buffer BM4 was added PAXgene RNA spin column and centrifuged for 15 seconds at 8000-20000x g. The flow-through was discarded. The spin column was placed in a new 2 ml processing tube and the old processing tube containing flow-through was discarded.
16. Another 500µl Buffer BM4 was added to the PAXgene RNA spin column and centrifuged for 2 minutes at 8000-20000 x g.
17. The processing tube containing flow through was discarded and the PAXgene RNA spin column was placed in a new 2 ml processing tube and centrifuged at 8000-20000 x g for 1 minute.
18. The processing tube containing flow-through was discarded. The PAXgene RNA spin column was placed in a new 1.5 ml microcentrifuged tube and 40µl Buffer BR5 was pipetted directly onto the spin column membrane. The lid was closed and centrifuged for 1 minute at 8000-20000 x g to elute the RNA.
19. The elution step 18 was repeated as described using 40µl Buffer BR5 and the same microcentrifuge tube.
20. The eluate was incubated for 5 minutes at 65°C in a shaker-incubator without shaking. After incubation, the sample was chilled immediately on ice.
21. Afterwards, the samples were ready for use, respectively storing.

### c) PAXgene Blood RNA Kit (PreAnalytiX)

The detailed protocol is described in the corresponding handbook. Thus, the respective protocol is only briefly described herein:
1. The PAXgene RNA tube was centrifuged for 10 minutes at 3000-5000 x g using a swing-out rotor.
2. The supernatant was removed by decanting or pipetting. 4 ml RNase-free water was added to the pellet and the tubes were closed.
3. The pellet was vortexed until visibly dissolved and centrifuged for 10 minutes at 3000-5000 x g using a swing-out rotor. The entire supernatant was removed and discarded.
4. 350µl resuspension buffer (BR1) was added and vortexed until the pellet was visibly dissolved.
5. The sample was pipetted into a 1.5 ml microecentrifuged tube. 300µl binding buffer (BR2) and 40µl proteinase K was added and mixed by vortexing for 5 seconds and incubated for 10 minutes at 55°C using a shaker-incubator at 400-1400 rpm. After incubation, the temperature of the shaker-incubator was set to 65°C for step 20.
6. The lysate was pipetted directly into a PAXgene Shredder spin column placed in 2 ml processing tube and centrifuged for 3 minutes at maximum speed.
7. The entire supernatant of the flow-through fraction was carefully transferred to a fresh 1.5 ml microcentrifuge tube without disturbing the pellet in the processing tube.
8. 350µl ethanol (96-100%, purity grade p.a.) was added and mixed by vortexing and centrifuged briefly (1-2 seconds at 500-1000 x g) to remove drops from the inside of the tube lid.
9. 700µl sample was pipetted into the PAXgene RNA spin column placed in a 2 ml processing tube and centrifuged for 1 minute at 8000-20000 x g. The spin column was placed in a new 2 ml processing tube and the old processing tube containing flow-through was discarded.
10. The remaining sample was pipetted into the PAXgene RNA spin column and centrifuged for 1 minute at 8000-20000 x g. The spin column was placed in a new 2 ml processing tube and the old processing tube containing flow-through was discarded.
11.350µl wash buffer 1 was pipetted into the PAXgene RNA spin column and centrifuged for 1 minute 8000-20000 x g. The spin column was placed in a new 2 ml processing tube and the old processing tube containing flow-through was discarded.
12. 10µl DNase I stock solution was added to 70µl DNA digestion buffer (RDD) in a 1.5 ml microcentrifuged tube, mixed and centrifuged briefly.
13. The DNase I incubation mix (80µl) was pipetted directly onto the PAXgene spin
   column membrane and placed on the benchtop for 15 minutes.
14.350µl wash buffer 1 (BR3) was pipetted into the PAXgene RNA spin column and
   centrifuged for 1 minute at 8000-20000 x g. The spin column was placed in a new 2 ml processing tube and the old processing tube containing flow-through was discarded.
15. 500µl wash buffer 2 (BR4) was pipetted into the PAXgene RNA spin column and centrifuged for 1 minute at 8000-20000 x g. The spin column was placed in a new 2 ml processing tube and the old processing tube containing flow-through was discarded.
16. Another 500µl wash buffer 2 (BR4) was added to the PAXgene RNA spin column and centrifuged for 3 minutes at 8000-20000 x g.
17. The processing tube containing the flow-through was discarded and the PAXgene RNA spin columns were placed in a new 2 ml processing tube and centrifuged for 1 minute at 8000-20000 x g.
18. The processing tube containing the flow-through was discarded and the PAXgene RNA spin columns were placed in an 1.5 ml centrifuged tube and 40µl elution buffer (BR5) was pipetted directly onto the PAXgene RNA spin column membrane and centrifuged for 1 minute at 8000-20000 x g to elute the RNA.
19. The elution step 18 was repeated as described, using 40µl elution buffer (BR5) and the same microcentrifuged tube.
20. The eluate was incubated for 5 minutes at 65°C in the shaker-incubator without shaking. After incubation, the sample was chilled immediately on ice.
21. The samples were now ready for use, respectively storing.

### d) Method according to the present invention

The method according to the present invention was performed on the QlAsymphony robotic system. In brief, the following steps were performed:
1. The PAXgene Blood RNA tube was centrifuged for 10min at 3.000 to 6.000rpm. The pellet was resuspended by the addition of a buffer (300µl) comprising ammonium acetate and vortexing.
2. Further lysis agents were added, preferably Proteinase K (40µl) and a lysis buffer (230µl) comprising more than 3M of a chaotropic salt, preferably GITC. Afterwards, the sample was incubated for 10min at 56°C.,
3. MagAttract beads were added to bind and remove DNA together with the silica particles.
4. For binding of the RNA, new MagAttract beads were added and 1500µl of a binding buffer according to the present invention comprising 80% isopropanol (or ethanol), 0,6M sodium perchlorate, sodium trifluoroacetate or sodium trichloroacetate respectively, and Tris.
5. Two washing steps were performed. The first washing step was performed with a buffer comprising isopropanol, GTC and a detergent; the second washing step was performed with a washing buffer comprising ethanol and Tris.
6. Afterwards, a DNase digest was performed.
7. For re-binding the RNA to the beads, 1400µl of the same binding buffer according to the present invention (see above) was added. This second binding step ensures a good yield of small RNAs.
8. Afterwards, four additional washing steps were performed.
9. Total RNA was eluted by using a suitable elution buffer (BR5, QIAGEN). Also other common elution buffers such as water or a Tris-buffer can be used.

### 2. Results

To analyse whether the small RNA was effectively isolated as part of the total RNA by the different compared methods, several miRNAs were detected using different established assays (see the figures for details). The CT values obtained with the eluates of each method are shown. The higher the CT value, the lower the concentration of the respective miRNA in the analysed eluate and accordingly, the less efficient was the isolation of small RNAs by the tested method.

The results show (see the figures) that the commercially available kits using magnetic silica particles are less efficient in isolating small RNAs than the methods of the present invention which use specific chaotropic salts for isolation. The best results are achieved with the manual PAXgene Blood miRNA kit, which, however has the drawback that it is silica membrane based and therefore is not suitable for a flexible, high throughput automated process. The results obtained with the methods according to the present inventions show that the method according to the present invention is suitable to isolate small RNAs with a good yield even if magnetic silica particles are used for isolation. Thus, the novel binding conditions provided by the present invention allow improving the standard RNA isolation methods that are based on the use of silica particles by using specific binding conditions, thereby allowing the efficient isolation of small RNAs even when using silica particles as nucleic acid binding carriers.

## Claims

1. A method for isolating small RNA from a sample, the method comprising binding RNA to silica particles by contacting the sample with
a) at least one alcohol,
b) at least one chaotropic salt comprising a chaotropic anion selected from the group consisting of trichloroacetate, perchlorate and trifluoroacetate and
c) silica particles
and separating the bound RNA from the rest of the sample.

2. The method according to claim 1, wherein the chaotropic anion is present during binding in a concentration of 0,05M to 2M, preferably 0,1M to less than 1M.

3. The method according to claim 1 or 2, wherein the chaotropic salt is selected from the group consisting of sodium perchlorate, sodium trichloroacetate and sodium trifluoroacetate, preferably sodium trichloroacetate.

4. The method according to any one of claims 1 to 3, wherein the alcohol is present during binding in a concentration of ≥ 40% v/v, preferably ≥ 40% v/v to ≤ 80% v/v, more preferred ≥ 50% v/v to ≤ 65% v/v.

5. The method according to any one of the claims 1 to 4, wherein the small RNA is obtained as a part of total RNA.

6. The method according to any one of claims 1 to 5, **characterised by** one or more of the following features:
i) the sample is a biological sample selected from body fluids, blood, blood products, tissue and bone marrow;
ii) the sample is mixed with a nucleic acid stabilisation composition for stabilising the nucleic acids; and/or
iii) the sample is mixed with a nucleic acid stabilization composition, wherein said stabilization composition comprises
a) a cationic compound of the general formula:
Y-R₁R₂R₃R₄X⁻
wherein Y represents nitrogen or phosphor, preferably nitrogen
R₁R₂R₃ and R₄ independently, represent a branched or unbranched C₁-C₂₀-alkyl group, a C₆-C₂₀-aryl group and/or a C₆-C₂₆ aralkyl group;
X⁻ represents an anion of an inorganic or organic, mono- or polybasic acid;
and
b) at least one proton donor.

7. The method according to any one of the claims 1 to 6, wherein the sample or a portion or part of the sample is pretreated prior to binding by the addition of at least one lysis agent, preferably a protease and/or a chaotropic agent.

8. The method according to any one of the claims 1 to 7, wherein after lysis, non-target nucleic acids are removed by the addition of a suitable nucleic acid binding carrier and under suitable conditions to bind at least a portion of the non-target nucleic acids.

9. The method according to any one of the claims 1 to 8, wherein the RNA bound to the silica particles is washed.

10. The method according to claim 9, wherein the RNA is washed at least once with one or more washing buffers, selected from
a) a washing buffer comprising a chaotropic agent and alcohol and preferably a detergent; and
b) a washing buffer comprising alcohol and a biological buffer.

11. The method according to claim 9, wherein after washing, the silica particles and the RNA are contacted again with at least one alcohol and a chaotropic salt comprising a chaotropic anion selected from the group consisting of trichloroacetate, perchlorate and trifluoroacetate, preferably under conditions as are defined in any one of claims 1 to 4.

12. A kit for isolating small RNA from a sample, comprising
a) optionally an alcohol,
b) at least one chaotropic salt comprising a chaotropic anion selected from the group consisting of trichloroacetate, perchlorate and trifluoroacetate and
c) silica particles.

13. The kit according to claim 12, comprising one of more of the following reagents:
a) isopropanol and/or ethanol;
b) a binding buffer comprising alcohol in a concentration of at least 60%, preferably at least 70% alcohol, at least 0,5M of a chaotropic salt comprising a chaotropic anion selected from the group consisting of trichloroacetate, perchlorate and trifluoroacetate, preferably sodium trichloroacetate and a biological buffer, preferably Tris;
c) magnetic silica particles;
d) a lysis buffer, preferably comprising a chaotropic agent;
e) a composition comprising a protease;
f) one or more washing buffers;
g) an elution buffer.

14. A composition for binding small RNA to silica particles comprising
a) at least one alcohol,
b) at least one chaotropic salt comprising a chaotropic anion selected from the group consisting of trichloroacetate, perchlorate and trifluoroacetate
c) silica particles
d) at least one sample or portion or part of a sample comprising nucleic acids.

15. The composition according to claim 14, having one or more of the following characteristics:
a) it comprises isopropanol and/or ethanol as alcohol, preferably isopropanol;
b) it comprises alcohol in a concentration of at least ≥ 40% v/v, preferably ≥ 40% v/v to ≤ 80% v/v, more preferred ≥ 50% v/v to ≤ 65% v/v;
c) it comprises the chaotropic anion in a concentration of 0,05M to 2M, preferably 0,1 M to less than 1M;
d) it comprises as chaotropic salt a salt selected from the group consisting of sodium trichloroacetate, sodium perchlorate and sodium trifluoroacetate, preferably sodium trichloroacetate;
e) it comprises a biological buffer, preferably Tris and/or
f) it comprises blood or portions or part of blood, comprising nucleic acids.
